# EUROPÄISCHE PATENTSCHRIFT

(11) **EP 0 758 872 B1**
(45) Veröffentlichungstag und Bekanntmachung des Hinweises auf die Patenterteilung: **06.05.1999**
(21) Anmeldenummer: 95908274.4
(22) Anmeldetag: 13.02.1995
(51) Int. Cl.: A61F 2/28, A61B 17/56, A61L 27/00

(54) **ABDECKEINRICHTUNG FÜR KNOCHENDEFEKTSTELLEN UND VERFAHREN ZU DEREN HERSTELLUNG**
COVERING SYSTEM FOR DEFECTIVE POINTS IN BONES AND PROCESS FOR PRODUCING IT
SYSTEME DE RECOUVREMENT DE POINTS ENDOMMAGES D'OS ET SON PROCEDE DE PRODUCTION

(30) Priorität: 27.04.1994 DE 4414675
(43) Veröffentlichungstag der Anmeldung: 26.02.1997
(73) Patentinhaber: Kirsch, Axel, Dr., 70794 Filderstadt (DE)
(72) Erfinder: Kirsch, Axel, Dr., 70794 Filderstadt (DE)
(74) Vertreter: Goddar, Heinz J., Dr.
(86) Internationale Anmeldenummer: EP9500519
(87) Internationale Veröffentlichungsnummer: WO9528900

(56) Entgegenhaltungen:
- EP-A- 0 338 976
- EP-A- 0 523 372
- EP-A- 0 525 210
- EP-A- 0 526 682
- WO-A-86/03667
- WO-A-88/03417
- WO-A-92/10218
- WO-A-94/03121
- DE-A- 4 302 708
- GB-A- 1 549 328

## Beschreibung

Die Erfindung betrifft eine Abdeckeinrichtung zum vorübergehenden Abdecken einer mit Knochenaufbaumaterial, wie Hydroxylapatitgranulat, gefüllte Knochendefektstelle, insbesondere Ausnehmung, in körpereigenem Knochengewebe, mit einem die Defektstelle im wesentlichen formstabil überdeckenden perforierten Versteifungselement aus verhältnismäßig steifem, jedoch biegsamem Material, wie Stützgitter aus Metall oder dergleichen, und einer durch das Versteifungselement getragenen ein- oder mehrschichtigen Abdeckmembran.

Ferner ist Gegenstand der Erfindung ein Verfahren zum Herstellen einer Abdeckeinrichtung zum vorübergehenden Abdecken einer mit Knochenaufbaumaterial, wie Hydroxylapatitgranulat, gefüllten Knochendefektstelle, insbesondere Ausnehmung, in körpereigenem Knochengewebe, bei dem ein die Defektstelle im wesentlichen formstabil überdeckendes perforiertes Versteifungselement aus verhältnismäßig steifem, jedoch biegsamem Material, wie Stützgitter aus Metall oder dergleichen, mit einer ein- oder mehrschichtigen Abdeckmembran versehen und in eine anwendungsspezifische Form gebracht wird.

In der Knochenchirurgie, beispielsweise bei der Rekonstruktion von Knochen in der plastischen Chirugie oder bei kieferchirugischen Operationen, ist es üblich, Knochendefektstellen in Form von Ausnehmungen oder Höhlungen im körpereigenen Knochengewebe mit Knochenaufbaumaterial zu füllen, welches in der Regel aus einer Mischung aus Knochenersatzmaterial, wie Hydroxylapatitgranulat, und körpereigenen Knochenpartikeln besteht. Um zu gewährleisten, daß das Knochenaufbaumaterial im wesentlichen ausschließlich von der Knochenseite her knöchern durchwachsen wird, nicht aber in unerwünschter Weise von Epithel und subepithelialem Bindegewebe, wird die Ausnehmung mit einer Abdeckmembran der eingangs genannten Art verschlossen. Nur dann nämlich, wenn ein vollständig knöchernes Durchwachsen des Knochenaufbaumaterials gewährleistet ist, läßt sich die Knochendefektstelle im wesentlichen vollständig beseitigen.

Bislang finden als Abdeckmembranen beispielsweise Polytetrafluorethylenfolien Verwendung, die nach Ausheilen der Knochendefektstelle entfernt werden müssen. Auch Folien aus resorbierbarem Material sind bekannt.

Infolge des für Abdeckmembranen verwendeten flexiblen Kunststoffmaterials ist die Membran nicht steif, sondern kann sich infolge einer mechanischen Beanspruchung durch das umgebende weiche Körpergewebe, z. B. infolge von Muskelbewegungen oder von außen einwirkenden Kräften, in ihrer Lage verändern. Derartige Mikrobewegungen der Abdeckmembran, die in allen Raumrichtungen erfolgen können, haben den Nachteil, daß das in der direkten Nachbarschaft der Abdeckmembran unter derselben befindliche knöchern prädeterminierte Granulationsgewebe, welches das primär entstehende Blutkoagel unter der Membran und zwischen den Partikeln des Knochenaufbaumaterials ersetzt, in der Weise dedifferenziert wird, daß sich subepitheliales faseriges Bindegewebe entwickelt.

Aus der DE 91 15 341 U1 ist ein Stützgitter zur Aufnahme von partikulären Knochenersatzmitteln bekannt, welches aus einem biologisch abbaubaren und thermoplastisch verformbaren Polymeren besteht. Das Stützgitter dient dazu, eine mechanische Abstützung von Knochenersatzmaterialien zu ermöglichen und beispielsweise bei vollständiger Durchtrennung des Unterkiefers die beiden Resektionsstümpfe gegeneinander zu fixieren. Ein Verschließen einer Knochenausnehmung gegenüber dem Nicht-Knochengewebe unter gleichzeitiger mechanischer Immobilisierung und Stabilisierung sowohl gegenüber dem umgebenden weichen Körpergewebe als auch gegenüber dem Knochenaufbaumaterial ist mittels dieses Stützgitters nicht möglich.

Aus der EP 0 504 103 A1 ist eine Befestigungseinrichtung für eine aus gewebeverträglicher Folie bestehende Abdeckmembran bekannt, jedoch treten bei einer derartigen beweglichen Abdeckmembran die bereits eingangs beschriebenen Probleme auf.

Gegenstand der älteren Patentanmeldung DE-A-43 02 709.1-35 ist eine Abdeckeinrichtung der gattungsgemäßen Art, mittels welcher die ungestörte Überführung des sich in der Knochendefektstelle bildernden knöchern prädeterminierten Granulationsgewebes im Knochengewebe dadurch gewährleistet ist, daß Bewegungen der Abdeckmembran durch das Versteifungselement verhindert werden. Das Versteifungselement, das in Anpassung an z. B. die Krümmung des Kieferknochens, in welchem sich die Knochendefektstelle befindet, geformt wird, sorgt dafür, daß das Blutkoagel ungestört in Knochengewebe überführt wird.

Bei der Abdeckeinrichtung der gattungsgemäßen Art wird die Abdeckmembran vor der anwendungsspezifischen Formgebung des Versteifungselementes mit diesem verbunden. Dies erfolgt vorzugsweise dadurch, daß werksseitig das Stützgitter bereits mit einer Abdeckmembran versehen wird.

Die vorstehend beschriebene Abdeckeinrichtung nach dem älteren Vorschlag hat sich in der Praxis durchaus bewährt, allerdings können Probleme insbesondere dann auftreten, wenn anwendungsspezifisch eine besonders starke Biegung oder dergleichen des Versteifungselementes notwendig ist, weil dann die vorher aufgebrachte Abdeckmembran leicht beschädigt werden kann, da sie, sofern sie die anwendungsspezifisch erforderliche Dicke hat, nicht ausreichend elastisch ist.

Der Erfindung liegt daher die Aufgabe zugrunde, die Abdeckeinrichtung und das Verfahren zu deren Herstellung der eingangs genannten Art dahingehend weiterzubilden, daß ein einwandfreies Anpassen der Abdeckeinrichtung an die Form der Knochendefektstelle und des sie umgebenden körpereigenen Knochens ohne Gefahr einer Beschädigung der Abdeckmembran auch unter ungünstigen Bedingungen gewährleistet ist.

Erfindungsgemäß wird diese Aufgabe in Weiterbildung der gattungsgemäßen Abdeckeinrichtung dadurch gelöst, daß mindestens eine der Schichten der Abdeckmembran als Lösungsfilm auf das Versteifungselement nach dessen anwendungsspezifischer Formgebung aufgebracht ist.

Dabei kann vorgesehen sein, daß das Versteifungselement auf der der Knochendefektstelle zu- und/oder abgewandten Fläche mit einer biaxial dehnbaren Basisfolie der Abdeckmembran versehen ist.

Die Erfindung schlägt gemäß einem Ausführungsbeispiel auch vor, daß der Lösungsfilm auf die Baisisfolie oder eine von mehreren Basisfolien der Abdeckmembran aufgebracht ist.

Nach einem Ausführungsbeispiel der Erfindung kann auch vorgesehen sein, daß in den Lösungsfilm der Abdeckmembran Hydroxylapatitpartikel oder dergleichen eingelagert sind.

Eine weitere Ausführungsform der Erfindung sieht vor, daß der Lösungsfilm der Abdeckmembran mindestens ein filmbildendes Material aus der Cyanacrylat, Kollagen, Polytetrafluorethylen, Polycarbonat, Polyurethan, Polylactid/Vicryl, Polylactid, Oxymethylcellulose und Methylmetacrylat enthaltenden Gruppe aufweist.

Das erfindungsgemäße Verfahren ist in Weiterbildung des Verfahrens der gattungsgemäßen Art dadurch gekennzeichnet, daß mindestens eine der Schichten der Abdeckmembran erst nach der anwendungsspezifischen Formgebung des Versteifungselementes auf dieses als Lösungsfilm aufgebracht wird.

Dabei kann vorgesehen sein, daß auf das Versteifungselement vor dem Aufbringen des Lösungsfilmes an der der Knochendefektstelle zu- und/oder abgewandten Seite eine biaxial dehnbare Basisfolie der Abdeckmembran aufgebracht wird.

Weiterhin sieht ein Ausführungsbeispiel der Erfindung vor, daß der Lösungsfilm auf das Versteifungselement unter Zwischenschaltung der Basisfolie oder mehrerer Basisfolien aufgelegt wird.

Es wird hierbei vorgesehen, daß das Versteifungselement bzw. die zur Aufnahme des Lösungsfilmes bestimmte Fläche der Basisfolie vor dem Aufbringen des Lösungsfilmes mit Hydroxylapatitpulver oder dergleichen belegt wird.

Dabei kann vorgesehen sein, daß zum Herstellen des Lösungsfilmes eine Lösung von lösungsfilmbildendem Material, welches mindestens einen Bestandteil aus der Cyanacrylat, Kollagen, Polytetrafluorethylen, Polycarbonat, Polyurethan, Polylactid/Vicryl, Polylactid, Oxymethylcellulose und Methylmetacrylat enthaltenden Gruppe aufweist, in organischem Lösungsmittel auf das Versteifungselement bzw. die Basisfolie der Abdeckmembran aufgebracht wird.

Die Erfindung schlägt in einen Ausführungsbeispiel auch vor, daß die Lösung auf das Versteifungselement bzw. die zur Aufnahme des Lösungsfilmes bestimmte Fläche der Basisfolie aufgesprüht wird.

Es ist dabei vorgesehen, daß die Lösung auf das Versteifungselement bzw. die zur Aufnahme des Lösungsmittels bestimmte Fläche der Basisfolie durch Aufstreichen, Aufpinseln oder dergleichen aufgebracht wird.

Der Erfindung liegt die überraschende Erkenntnis zugrunde, daß es gelingt, die bei der Anwendung der gattungsgemäßen Abdeckeinrichtung in bestimmten Anwendungsfällen noch bestehenden Schwierigkeiten dadurch zu beheben, daß dem Versteifungselement, wie Stützgitter aus Titan oder dergleichen, zunächst die anwendungsspezifisch notwendige Form gegeben wird, ehe dann die Abdeckmembran, bzw. wenigstens eine der Schichten, vorzugsweise die obere Schicht, der Abdeckmembran aufgebracht wird. Das Aufbringen der Abdeckmembran erfolgt dann nach Art eines Wundsprühpflasters durch Bildung eines Lösungsfilmes, wobei gegebenenfalls auch ein Polymerisationsvorgang eingeschlossen sein kann.

Bei der Anwendung der Erfindung kann dann entweder so vorgegangen werden, daß dem Versteifungselement vor dem Anbringen an der Knochendefektstelle bereits die endgültige Form gegeben wird, z. B. unter Verwendung eines Modells oder einer Schablone, ehe die Abdeckmembran durch Lösungsfilmbildung aufgebracht wird, jedoch kann natürlich in besonders günstiger Weise auch so vorgegangen werden, daß zunächst das Anbringen des Versteifungselementes an der Knochendefektstelle, unter endgültiger Formgebung, erfolgt, ehe dann wundsprühpflasterartig die Abdeckmembran bzw. mindestens eine Schicht derselben als Lösungsfilm aufgebracht wird.

Besonders günstig ist es dabei, daß, wie dies eine Ausführungsform der Erfindung vorsieht, in dem Lösungsfilm der Abdeckmembran Hydroxylapatitpulver oder dergleichen eingebettet werden kann, wodurch der Einwachsprozess besonders gefördert wird. Natürlich ist es auch möglich, ein Versteifungselement zu verwenden, welches bereits vor dem Aufsprühen des Lösungsfilmes ein- oder beidseitig mit einer dünnen Basisfolie der Abdeckmembran versehen worden ist, wobei für diese Basisfolie, ebenso wie für den Lösungsfilm der Abdeckmembran, aus dem dieselbe selbstverständlich auch gänzlich bestehen kann, Materialien geeignet sind, wie sie Gegenstand der älteren Patentanmeldung DE-A-43 02 709.1-35 sind, auf die insoweit zur weiteren Erläuterung des Erfindungsgedankens in vollem Umfang Bezug genommen wird.

Weitere Merkmale und Vorteile der Erfindung ergeben sich aus den Ansprüchen und aus der nachstehenden Beschreibung, in der Ausführungsbeispiele anhand der schematischen Zeichnung im einzelnen erläutert sind. Dabei zeigt:
- Fig. 1: eine mit einer Abdeckeinrichtung nach einem Ausführungsbeispiel gemäß der Erfindung abgedeckte Knochendefektstelle im Schnitt senkrecht durch die Ebene der Abdeckeinrichtung;
- Fig. 2: das Versteifungselement der Abdeckeinrichtung gemäß Fig. 1 im Schnitt senkrecht zur Ebene der Abdeckmembran, in vergrößerter Darstellung;
- Fig. 3: ein weiteres Ausführungsbeispiel der Abdeckmembran von Fig. 1 im Schnitt senkrecht zur Ebene der Abdeckeinrichtung in vergrößerter Darstellung;
- Fig. 4: ein drittes Ausführungsbeispiel einer Abdeckeinrichtung nach der Erfindung im Schnitt senkrecht zur Ebene der Abdeckeinrichtung; und
- Fig. 5: ein viertes Ausführungsbeispiel einer Abdeckeinrichtung nach der Erfindung im Schnitt senkrecht zur Ebene der Abdeckeinrichtung.

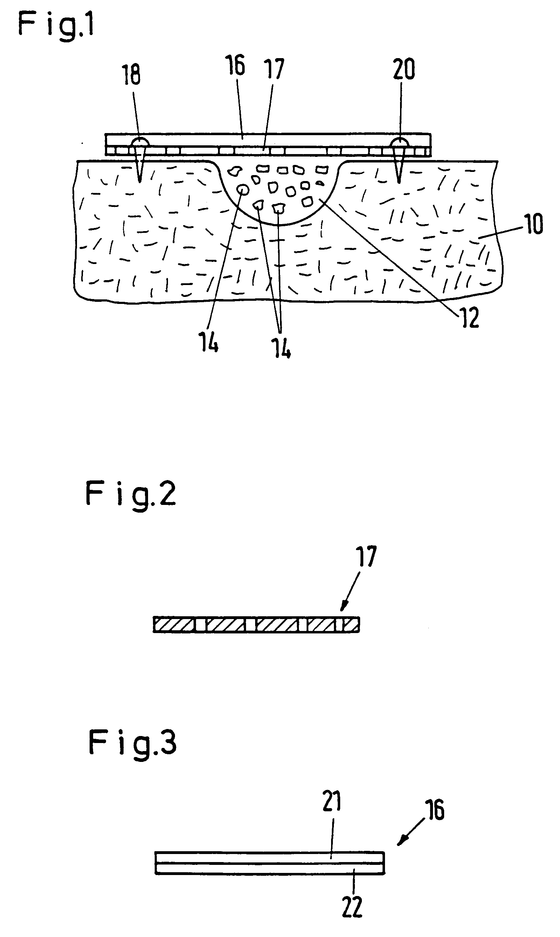

Wie Fig. 1 erkennen läßt, ist dort eine in einem körpereigenen Knochen 10 durch eine Ausnehmung 12 gebildete Knochendefektstelle mit Knochenaufbaumaterial 14 aus Hydroxylapatitgranulat im wesentlichen vollständig gefüllt, wobei diesem Hydroxylapatitgranulat in bekannter Weise aus körpereigenem Knochengewebe bestehende Knochenpartikel beigemengt sind. Die mit dem Knochenaufbaumaterial 14 gefüllte Ausnehmung 12 ist von einer Abdeckmembran 16 abgedeckt, die sich an der dem Knochen 10 abgewandten Seite eines Versteifungselementes 17, bei dem es sich um ein perforiertes dünnes Titanblech handelt, angeordnet ist. Die Abdeckmembran 16 und das Versteifungselement 17 sind allseits der Ausnehmung 12 mittels Befestigungsnägeln 18, 20 in dichter Anlage am körpereigenen Knochen 10 befestigt. Die Abdeckmembran 16 hat den Zweck, das knöcherne Durchwachsen des Knochenaufbaumaterials 14 vom körpereigenen Knochen 10 her in der Weise zu gewährleisten, daß das Durchwachsen des Knochenaufbaumaterials mit anderem als Knochengewebe, insbesondere Schleimhautgewebe, verhindert wird.

Bei dem Ausführungsbeispiel von Fig. 1 ist die Abdeckmembran 16 als einschichtiger Lösungsfilm aus atmungsaktivem Methylmethacrylat ausgebildet, der nach der endgültigen Formgebung des Versteifungselementes 17 auf dieses aufgebracht worden ist.

In Fig. 2 ist in vergrößerter Darstellung das Versteifungselement 17, aus perforiertem Titanblech bestehend, wiedergegeben, welches bei dem Ausführungsbeispiel von Fig. 1 Verwendung findet und auf welches dann die Abdeckmembran 16 als Lösungsfilm aufgebracht worden ist.

Beim Ausführungsbeispiel von Fig. 3 besteht die Abdeckmembran 16 aus einer Basisfolie 22 aus Oxymethylcellulose, die so dünn ist, daß sie biaxial leicht verformt werden kann, wobei also die Basisfolie 22 zunächst noch vor dem Verformen des zugehörigen Versteifungselementes 17 auf dieses aufgebracht wird. Bei der Formgebung des Versteifungselementes 17 wird die Basisfolie 22 entsprechend biaxial verstreckt, ohne zu reißen. Auf das endgültig geformte Versteifungselement wird dann ein Lösungsfilm 21 aufgebracht.

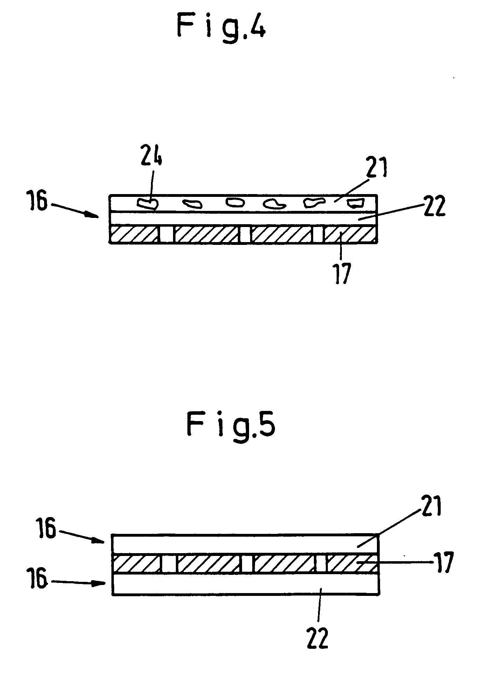

Beim Ausführungsbeispiel von Fig. 4 trägt das Versteifungselement 17 zunächst die bereits beim Ausführungsbeispiel von Fig. 3 vorhandene Basisfolie 22, worauf sich dann der Lösungsfilm 21 anschließt, in den Hydroxylapatitpartikel 24 eingebettet sind.

Beim Ausführungsbeispiel von Fig. 5 befindet sich die Basisfolie 22 an der in der Zeichnung unten liegenden, dem Knochen zugewandten Seiten des Versteifungselementes 17, während sich der Lösungsfilm 21 auf der dem in Fig. 5 nicht gezeigten Knochen abgewandten Seite des Versteifungselementes 17 befindet.

Die Herstellung der Abdeckeinrichtung gemäß Fig. 1 erfolgt in der Weise, daß nach der endgültigen Formgebung des Versteifungselementes 17 auf dieses eine Methacrylsäuremethylesterlösung nach Art der Herstellung eines Wundpflastersprühverbandes aufgesprüht wird. Nach dem Verdunsten des organischen Lösungsmittels ergibt sich dann der Lösungsfilm 21.

Beim Ausführungsbeispiel von Fig. 3 wird in der Weise vorgegangen, daß die Lösung des lösungsmittelbildenden Materials auf die Basisfolie 22 aufgesprücht wird.

Beim Ausführungsbeispiel von Fig. 4 wird derart vorgegangen, daß die dem Versteifungselement 17 abgewandte Fläche der Basisfolie 22 zunächst mit Hydroxylapatitpulver bestäubt wird.

Anschließend wird dann der Lösungsfilm 21 aufgebracht, wiederum durch Aufsprühen, wodurch dann die Hydroxylapatitpartikel 24 in den Lösungsfilm 21 eingebettet werden.

Beim Ausführungsbeispiel von Fig. 5 ist die Vorgehensweise derart, daß die Lösung des lösungsmittelbildenden Materials auf das Versteifungselement 17 an der der Basisfolie 22 abgewandten Seite aufgepinselt wird, wobei sich dann durch Verdunsten des organsichen Lösungsmittels der Lösungsfilm 21 bildet.

## Patentansprüche

1. Abdeckeinrichtung zum vorübergehenden Abdecken einer mit Knochenaufbaumaterial (14), wie Hydroxylapatitgranulat, gefüllten Knochendefektstelle (12), insbesondere Ausnehmung, in körpereigenem Knochengewebe (10), mit einem die Defektstelle (12) im wesentlichen formstabil überdeckenden perforierten Versteifungselement (17) aus verhältnismäßig steifem, jedoch biegsamem Material, wie Stützgitter aus Metall oder dergleichen, und einer durch das Versteifungselement (17) getragenen ein- oder mehrschichtigen Abdeckmembran (16), dadurch gekennzeichnet, daß mindestens eine der Schichten (21, 22) der Abdeckmembran (16) als Lösungsfilm auf das anwendungsspezifisch geformte Versteifungselement (17) aufgebracht ist.

2. Abdeckeinrichtung nach Anspruch 1, dadurch gekennzeichnet, daß das Versteifungselement (17) auf der der Knochendefektstelle (12) zu- und/oder abgewandten Fläche mit einer biaxial dehnbaren Basisfolie (22) der Abdeckmembran (16) versehen ist.

3. Abdeckeinrichtung nach Anspruch 2, dadurch gekennzeichnet, daß der Lösungsfilm (21) auf die Basisfolie oder eine von mehreren Basisfolien (22) der Abdeckmembran (16) aufgebracht ist.

4. Abdeckeinrichtung nach einem der vorangehenden Ansprüche, dadurch gekennzeichnet, daß in den Lösungsfilm (21) der Abdeckmembran (16) Hydroxylapatitpartikel (24) oder dergleichen eingelagert sind.

5. Abdeckeinrichtung nach einem der vorangehenden Ansprüche, dadurch gekennzeichnet, daß der Lösungsfilm (21) der Abdeckmembran (16) mindestens ein filmbildendes Material aus der Cyanacrylat, Kollagen, Polytetrafluorethylen, Polycarbonat, Polyurethan, Polylactid/Vicryl, Polylactid, Oxymethylcellulose und Methylmetacrylat enthaltenden Gruppe aufweist.

6. Verfahren zum Herstellen einer Abdeckeinrichtung zum vorübergehenden Abdecken einer mit Knochenaufbaumaterial (14), wie Hydroxilapatitgranulat, gefüllten Knochendefektstelle (12), insbesondere Ausnehmung, in körpereigenem Knochengewebe (10), bei dem ein die Defektstelle im wesentlichen formstabil überdeckendes perforiertes Versteifungselement (17) aus verhältnismäßig steifem, jedoch biegsamem Material, wie Stützgitter aus Metall oder dergleichen, mit einer ein- oder mehrschichtigen Abdeckmembran (16) versehen und in eine anwendungsspezifische Form gebracht wird, insbesondere zur Herstellung einer Abdeckeinrichtung nach einem der vorangehenden Ansprüche, dadurch gekennzeichnet, daß mindestens eine der Schichten der Abdeckmembran (16) erst nach der anwendungsspezifischen Formgebung des Versteifungselementes auf dieses als Lösungsfilm aufgebracht wird.

7. Verfahren nach Anspruch 6, dadurch gekennzeichnet, daß auf das Versteifungselement (17) vor dem Aufbringen des Lösungsfilmes an der der Knochendefektstelle (12) zu- und/oder abgewandten Seite eine biaxial dehnbare Basisfolie (22) der Abdeckmembran aufgebracht wird.

8. Verfahren nach Anspruch 7, dadurch gekennzeichnet, daß der Lösungsfilm auf das Versteifungselement (17) unter Zwischenschaltung der Basisfolie(22) oder mehrerer Basisfolien (22) aufgelegt wird.

9. Verfahren nach einem der Ansprüche 6 bis 8, dadurch gekennzeichnet, daß das Versteifungselement (17) bzw. die zur Aufnahme des Lösungsfilmes bestimmte Fläche der Basisfolie (22) vor dem Aufbringen des Lösungsfilmes mit Hydroxylapatitpulver oder dergleichen belegt wird.

10. Verfahren nach einem der Ansprüche 6 bis 9, dadurch gekennzeichnet, daß zum Herstellen des Lösungsfilmes eine Lösung von lösungsfilmbildendem Material, welches mindestens einen Bestandteil aus der Cyanacrylat, Kollagen, Polytetrafluorethylen, Polycarbonat, Polyurethan, Polylactid/Vicryl, Polylactid, Oxymethylcellulose und Methylmetacrylat enthaltenden Gruppe aufweist, in organischem Lösungsmittel auf das Versteifungselement bzw. die Basisfolie der Abdeckmembran aufgebracht wird.

11. Verfahren nach Anspruch 10, dadurch gekennzeichnet, daß die Lösung auf das Versteifungselement (17) bzw. die zur Aufnahme des Lösungsfilmes bestimmte Fläche der Basisfolie (22) aufgesprüht wird.

12. Verfahren nach Anspruch 10, dadurch gekennzeichnet, daß die Lösung auf das Versteifungselement (17) bzw. die zur Aufnahme des Lösungsmittels bestimmte Fläche der Basisfolie (22) durch Aufstreichen, Aufpinseln oder dergleichen aufgebracht wird.

## Claims

1. A covering system whereby a bone defect (12), more particularly a recess in the endogenous bone tissue (10) filled with bone construction material (14) such as hydroxyl apatite granulate is temporarily covered by a perforated stiffening element (17) made of relatively stiff but flexible material, such as a metal or similar supporting grid covering the defect (12) in substantially dimensionally stable manner, and a single-layer or multi-layer covering membrane (16) supported by the stiffening element (17), characterised in that at least one of the layers (21, 22) of the covering membrane (16) is applied in the form of a film of solution to the stiffening element (17) shaped for the specific application.

2. A covering system according to claim 1, characterised in that the stiffening element (17) is lined with a biaxially stretchable base sheet (22) of the covering membrane (16) on the surface facing and/or remote from the bone defect (12).

3. A covering system according to claim 2, characterised in that the film (21) of solution is applied to the base sheet or to one out of a number of base sheets (22) of the covering membrane (16).

4. A covering system according to any of the preceding claims, characterised in that hydroxyl apatite particles (24) or the like are incorporated in the solution film (21) of the covering membrane (16).

5. A covering system according to any of the preceding claims, characterised in that the solution film (21) of the covering membrane (16) comprises at least one film-forming material out of the group containing cyanoacrylate, collagen, polytetrafluoroethylene, polycarbonate, polyurethane, polylactide/vicryl, polylactide, oxymethyl cellulose and methyl methacrylate.

6. A method of producing a covering system for temporarily covering a bone defect (12), more particularly a recess in the endogenous bone tissue (10) filled with bone construction material (14) such as hydroxyl apatite granulate, wherein a perforated stiffening element (17) covering the defect in substantially dimensionally stable manner and made of relatively stiff but flexible material, such as a metal or similar supporting grid, is covered with a single-layer or multi-layer membrane (16) and converted into a shape for the specific application, more particularly for producing a covering system according to any of the preceding claims, characterised in that at least one layer of the covering membrane (16) is not applied as a solution film to the stiffening element until the element has been shaped for its specific purpose.

7. A method according to claim 6, characterised in that before the solution film is applied, the stiffening element (17) is lined with a biaxially stretchable base sheet (22) of the covering membrane on the side facing and/or remote from the bone defect (12).

8. A method according to claim 7, characterised in that the solution film is applied to the stiffening element (17) with interposition of the base sheet (22) or a number of base sheets (22).

9. A method according to any of claims 6 to 8, chracterised in that the stiffening element (17) or the surface of the base foil (22) for receiving the solution film is coated with hydroxyl apatite powder or the like before the solution film is applied.

10. A method according to any of claims 6 to 9, characterised in that in order to produce the solution film, a solution of solution film-forming material comprising at least one constituent from the group containing cyanoacrylate, collagen, polytetrafluoroethylene, polycarbonate, polyurethane, polylactide/vicryl, polylactide, oxymethyl cellulose and methyl methacrylate is applied in organic solvent to the stiffening element or the base sheet of the cover membrane.

11. A method according to claim 10, characterised in that the solution is sprayed on to the stiffening element (17) or on to the surface of the base sheet (22) for receiving the solution film.

12. A method according to claim 10, characterised in that the solution is applied by spreading, brushing or the like to the stiffening element (17) or to the surface of the base sheet (22) for receiving the solvent.

## Revendications

1. Dispositif de recouvrement pour recouvrir temporairement un endroit endommagé (12) d'un os, notamment un creux, rempli de matière structurelle osseuse (14), par exemple de granulat d'apatite hydroxylique, dans le tissu osseux (10) d'un corps, avec un élément de renfort (17) perforé, recouvrant en une forme globalement stable l'endroit endommagé (12) et construit en un matériau relativement solide mais flexible, comme une grille de soutien en métal ou en un matériau analogue, et avec une membrane recouvrante (16) à une ou plusieurs couches et portée par l'élément de renfort (17), caractérisé en ce qu'au moins l'une des couches (21, 22) de la membrane recouvrante (16) est appliquée comme un film de solution sur l'élément de renfort (17) formé de manière spécifique à l'utilisation.

2. Dispositif de recouvrement selon la revendication 1, caractérisé en ce que l'élément de renfort (17) est muni, sur la surface éloignée et/ou sur la surface proche de l'endroit endommagé (12) de l'os, d'une feuille de base (22), extensible selon les deux axes, de la membrane recouvrante (16).

3. Dispositif de recouvrement selon la revendication 2, caractérisé en ce que le film de solution (21) est appliqué sur la feuille de base ou sur une parmi plusieurs feuilles de base (22) de la membrane recouvrante (16).

4. Dispositif de recouvrement selon l'une des revendications précédentes, caractérisé en ce qu'il est intercalé dans le film de solution (21) de la membrane recouvrante (16) des particules d'apatite hydroxylique (24) ou analogues.

5. Dispositif de recouvrement selon l'une des revendications précédentes, caractérisé en ce que le film de solution (21) de la membrane recouvrante (16) comporte au moins un matériau formant le film et du groupe contenant cyanoacrylate, collagène, polytétrafluoroéthylène, polycarbonate, polyuréthane, polylactide/vicryle, polylactide, cellulose oxyméthylique et méthacrylate de méthyle.

6. Procédé de fabrication d'un dispositif de recouvrement pour recouvrir temporairement un endroit endommagé (12) d'un os, notamment un creux, rempli de matière structurelle osseuse (14), par exemple de granulat d'apatite hydroxylique, dans le tissu osseux (10) d'un corps, dans lequel un élément de renfort (17) perforé, recouvrant en une forme globalement stable l'endroit endommagé (12) et construit en un matériau relativement solide mais flexible, comme une grille de soutien en métal ou en un matériau analogue, est muni d'une membrane recouvrante (16) à une ou plusieurs couches et reçoit une forme spécifique à l'utilisation, notamment pour la fabrication d'un dispositif de recouvrement selon l'une des revendications précédentes, caractérisé en ce que l'on applique comme film de solution sur l'élément de renfort au moins l'une des couches de la membrane recouvrante (16) seulement après avoir donné à cet élément de renfort sa forme spécifique à l'utilisation.

7. Procédé selon la revendication 6, caractérisé en ce que l'on applique sur l'élément de renfort (17), avant l'application du film de solution, sur le côté éloigné et/ou sur le côté proche de l'endroit endommagé (12) de l'os une feuille de base (22), extensible selon les deux axes, de la membrane recouvrante.

8. Procédé selon la revendication 7, caractérisé en ce que l'on applique le film de solution sur l'élément de renfort (17) en intercalant la feuille de base (22) ou plusieurs feuilles de base (22).

9. Procédé selon l'une des revendications 6 à 8, caractérisé en ce que, avant l'application du film de solution, on couvre l'élément de renfort (17), c'est-à-dire la surface, destinée à recevoir le film de solution, de la feuille de base (22), d'une poudre d'apatite hydroxylique ou analogue.

10. Procédé selon l'une des revendications 6 à 9, caractérisé en ce que, pour fabriquer le film de solution, on applique sur l'élément de renfort, c'est-à-dire sur la feuille de base de la membrane recouvrante, une solution dans un solvant organique d'un matériau formant le film de solution et comportant au moins une partie du groupe contenant cyanoacrylate, collagène, polytétrafluoroéthylène, polycarbonate, polyuréthane, polylactide/vicryle, polylactide, cellulose oxyméthylique et méthacrylate de méthyle.

11. Procédé selon la revendication 10, caractérisé en ce que l'on asperge avec la solution l'élément de renfort (17), c'est-à-dire la surface, destinée à recevoir le film de solution, de la feuille de base (22).

12. Procédé selon la revendication 10, caractérisé en ce que l'on applique la solution sur l'élément de renfort (17), c'est-à-dire sur la surface, destinée à recevoir le film de solution, de la feuille de base (22), en l'enduisant à la racle, en le badigeonnant au pinceau ou en une autre opération analogue.
